# EUROPÄISCHE PATENTSCHRIFT

(11) **EP 3 827 762 B1**
(45) Veröffentlichungstag und Bekanntmachung des Hinweises auf die Patenterteilung: **07.06.2023**
(21) Anmeldenummer: 20209129.4
(22) Anmeldetag: 23.11.2020
(51) Int. Cl.: A61B 17/221

(54) **MEDIZINISCHE VORRICHTUNG ZUR INTRAVASKULÄREN BEHANDLUNG UND HERSTELLUNGSVERFAHREN**
MEDICAL DEVICE FOR INTRAVASCULAR TREATMENT AND METHOD OF MANUFACTURING THE SAME
DISPOSITIF MÉDICAL DESTINÉ AU TRAITEMENT INTRAVASCULAIRE ET PROCÉDÉ DE FABRICATION

(30) Priorität: 28.11.2019 DE 102019132295; 02.04.2020 DE 102020109158
(43) Veröffentlichungstag der Anmeldung: 02.06.2021
(73) Patentinhaber: Acandis GmbH, 75177 Pforzheim (DE)
(72) Erfinder: LANGE, Alexander, 76135 Karlsruhe (DE)
(74) Vertreter: Meissner Bolte Partnerschaft mbB

(56) Entgegenhaltungen:
- CN-A- 108 042 176
- DE-A1-102018 107 594
- US-A1- 2013 211 492
- US-A1- 2014 088 678
- US-A1- 2014 194 911
- US-A1- 2014 343 595
- US-A1- 2019 000 492
- US-A1- 2019 239 907

## Beschreibung

Die Erfindung betrifft eine medizinische Vorrichtung zur intravaskulären Behandlung gemäß dem Oberbegriff des Patentanspruchs 1 sowie ein Herstellungsverfahren. Eine medizinische Vorrichtung dieser Art ist beispielsweise aus CN 108 042 176 A bekannt.

Das aus CN 108 042 176 A weist eine Gitterstruktur auf, die mit einem Transportdraht formschlüssig gekoppelt ist. Dazu ist an einem Stegfortsatz der Gitterstruktur eine gezahnte Struktur mit Erhebungen und Nuten vorgesehen, die sich quer zur Längsrichtung des Stegfortsatzes erstrecken. Ein korrespondierendes Gegenstück ist am Transportdraht angebracht. Zur Verbindung greifen die Erhebungen am Gegenstück des Transportdrahts in die Nuten der gezahnten Struktur am Stegfortsatz ein. Um die Verbindung auch quer zur Längsrichtung des Transportdrahtes bzw. Stegfortsatzes zu sichern, ist eine Sicherungshülse vorgesehen, die die gezahnte Struktur und das Gegenstück umgreift.

Diese bekannte Verbindung zwischen Gitterstruktur und Transportdraht hat mehrere Nachteile. Einerseits erhöht die Sicherungshülse den Querschnittsdurchmesser an der Verbindungsstelle, was die Zuführbarkeit des Trombektomiedevices in kleine Blutgefäß erschwert. Andererseits ist die Sicherungshülse hohen Kräften ausgesetzt, insbesondere bei einer Torsion zwischen der Gitterstruktur und dem Transportdraht. Eine solche Torsion kann auftreten, wenn sich die gerollte Gitterstruktur im Blutgefäß entfaltet bzw. "entrollt". Die Sicherungshülse kann dabei Schaden nehmen, so dass die Gefahr besteht, dass sich die Gitterstruktur vom Transportdraht löst. Ebenso kann die Hülse durch Biegekräfte beschädigt werden, die beim Führen des Transportdrahts durch enge Blutgefäße entstehen können. Wenn die Biegekräfte eine Kraftkomponente haben, die in Richtung der Nuten bzw. Erhebungen verläuft, kann es zu einer Verschiebung von Transportdraht und Stegfortsatz kommen, wodurch die Sicherungshülse Scherkräften ausgesetzt wird.

Aus DE 10 2018 107 594 A1 ist ein Thrombektomie-Device, auch als Stent Retriever bezeichnet, bekannt, das zur schnellen Rekanalisation geeignet ist und ein hybrides Zelldesign aufweist. Dadurch wird eine hervorragende Wandapposition und Thrombus-Integration erreicht.

Das Thrombektomie-Device besteht aus einer rohrförmigen Gitterstruktur, bei der Stege einzelne Zellen begrenzen. Derartige Gitterstrukturen sind üblicherweise aus einem Formgedächtnismaterial, wie beispielsweise Nitinol, lasergeschnitten.

Das proximale Ende der Gitterstruktur ist mit einem distalen Ende eines Transportdrahts verbunden. Dabei besteht die Verbindung aus einer formschlüssigen Verbindung, bei der die Enden der zu fügenden Komponenten (Stentkomponente und Transportdraht) ineinander verzahnt zusammengefügt und durch eine die Fügestelle umgreifende Crimphülse gesichert werden.

Die in DE 10 2018 107 594 A1 beschriebene formschlüssige Verbindung hat sich in der bisherigen Praxis für den Einsatz medizinischer Vorrichtungen in 0,021" Kathetern mit einem Innenlumen von 0,530 mm bewährt. Jedoch weisen medizinische Vorrichtungen mit einer derartigen formschlüssigen Verbindung eine beschränkte Einsetzbarkeit auf.

Die Erfindung liegt die Aufgabe zu Grunde, eine medizinische Vorrichtung zur intravaskulären Behandlung anzugeben, dessen Einsatzmöglichkeiten erweitert sind, ohne dass dabei die Funktionssicherheit der Vorrichtung wesentlich beeinträchtigt wird. Ferner besteht die Aufgabe der Erfindung darin, ein Verfahren zur Herstellung einer derartigen Vorrichtung anzugeben.

Erfindungsgemäß wird diese Aufgabe im Hinblick auf die medizinische Vorrichtung durch den Gegenstand des Patentanspruchs 1 und im Hinblick auf das Herstellungsverfahren durch den Gegenstand des Patentanspruchs 13 gelöst.

Konkret wird die Aufgabe durch eine medizinische Vorrichtung zur intravaskulären Behandlung mit einer zumindest abschnittsweise rohrförmigen Gitterstruktur aus einstückig miteinander verbundenen Stegen gelöst, wobei die Gitterstruktur an einem proximalen Endabschnitt einen Stegfortsatz aufweist, der ein Eingriffselement umfasst. Das Eingriffselement ist durch eine formschlüssige Verbindung mit einem distalen Kopplungsstück eines Transportdrahts verbunden.

Erfindungsgemäß ist vorgesehen, dass das Kopplungsstück wenigstens einen Vorsprung und das Eingriffselement wenigstens eine Ausnehmung aufweisen, wobei der Vorsprung in die Ausnehmung des Eingriffselements eingreift.

Der Vorteil der Erfindung besteht darin, dass aufgrund des formschlüssigen Eingreifens des Vorsprungs in die Ausnehmung des Eingriffselements eine ausreichend starke Verbindung zwischen der Stentkomponente und dem Transportdraht geschaffen wird, sodass keine zusätzliche Sicherung direkt an der Verbindungsstelle selbst erforderlich ist. Die starke Verbindung ermöglicht es, mögliche zusätzliche Sicherungselemente zur Fixierung der Verbindungsstelle flexibel anzuordnen.

Im Allgemeinen wird an dieser Stelle darauf hingewiesen, dass die medizinische Vorrichtung eine rohrförmige Gitterstruktur aufweist, die radial expandierbar ist. Dabei kann die rohrförmige Gitterstruktur selbsttätig, beispielsweise unter Ausnutzung des Shape-Memory-Effekts, von einem radial komprimierten in einen radial expandierten Zustand überführt werden. Insofern weist die medizinische Vorrichtung vorzugsweise eine selbstexpandierende Gitterstruktur auf. Die Gitterstruktur kann abschnittsweise oder vollständig rohrförmig ausgebildet sein.

Bei einer Ausgestaltung der medizinischen Vorrichtung als Thrombektomie-Device kann in bevorzugter Weise vorgesehen sein, dass die Gitterstruktur abschnittsweise rohrförmig ausgebildet ist, wobei die Rohrform an einem proximalen Ende der Gitterstruktur in einem trichterförmigen bzw. kegelförmigen Abschnitt übergeht.

Ferner wird darauf hingewiesen, dass im Rahmen der vorliegenden Patentanmeldung der Begriff "distal" eine vom Benutzer der Vorrichtung abgewandte Seite bzw. entfernt liegende Seite der Gitterstruktur bedeutet. Der Begriff "proximal" bedeutet eine zum Benutzer hinweisende Seite der medizinischen Vorrichtung, d.h. eine näher am Benutzer liegende Seite.

Erfindungsgemäß ist die Ausnehmung geschlossen ausgebildet, wobei sich die Ausnehmung im Wesentlichen in axialer Richtung des Eingriffselements erstreckt. Durch die geschlossene Ausbildung der Ausnehmung stehen der Vorsprung und die Ausnehmung über den gesamten Umfang in Eingriff, sodass dadurch größere Kräfte übertragbar sind.

Um eine hohe Gestaltungsvielfalt bei der Herstellung der medizinischen Vorrichtung zu erreichen, entspricht die Ausnehmung vorzugsweise im Wesentlichen der Kontur des Eingriffselements. Beispielsweise ist vorgesehen, dass das Eingriffselement eine rechteckige Kontur aufweist, sodass die Ausnehmung ebenfalls eine rechteckige Kontur aufweist, wobei die Ausnehmung wenigstens zwei Seiten umfasst, die gegenüber angeordnet sind.

In einer bevorzugten Ausführungsform weist das Kopplungsstück wenigstens zwei Vorsprünge auf, die als Queranker ausgebildet sind. Durch die Vorsprünge ist das proximale Ende der Gitterstruktur mit dem distalen Ende des Transportdrahts, genauer gesagt, das Eingriffselement mit dem Kopplungsstück formschlüssig verbunden bzw. verbindbar. Ein Vorsprung reicht aus, um eine formschlüssige Verbindung sicherzustellen. Die Ausbildung wenigstens zweier Vorsprünge in Form eines Querankers bringt den Vorteil mit sich, dass eine noch sichere Verbindung der Gitterstruktur mit dem Transportdraht geschaffen und eine symmetrische Krafteinleitung ermöglicht wird. Dadurch ist die Verbindung ausreichend stark, um die auftretenden Kräfte übertragen zu können. Diese Ausführungsform ist besonders gut als Thrombektomie-Device geeignet.

Während der Behandlung, insbesondere wenn die medizinische Vorrichtung nach einem Positionierungsversuch wieder in ein Zuführsystem, beispielsweise einen Katheter, zurückgezogen werden soll, treten Kräfte insbesondere Zug- und Druckkräfte in axialer Richtung bezogen auf das Kopplungsstück sowie Biege- und Torsionskräfte auf. Um eine gleichmäßige, insbesondere symmetrische Übertragung dieser auftretenden Kräfte zu ermöglichen, und zwar derart, dass auch eine ausreichend sichere axiale Fixierung des Kopplungsstücks sichergestellt ist, ist der Queranker vorzugsweise als T-Anker ausgebildet. Des Weiteren lässt sich ein T-Anker einfach herstellen.

In einer weiteren bevorzugten Ausführungsform entspricht der Vorsprung der Form der Ausnehmung. Das bedeutet, dass die Ausnehmung im Wesentlichen dieselbe Form wie der Vorsprung aufweist, insbesondere die entsprechende Negativform zeigt. Dies hat den Vorteil, dass der Vorsprung passgenau in die Ausnehmung angeordnet werden kann. So kann der Transportdraht nahezu jeder Stentstruktur angepasst werden, sodass dadurch eine flexible bzw. hohe Gestaltungsvielfalt bei der Herstellung der medizinischen Vorrichtung erreicht wird.

Grundsätzlich kann sich die Ausnehmung sowohl in axialer als auch in vertikaler Richtung des Eingriffselements erstrecken. Da die größten Kräfte jedoch in axialer Richtung bezogen auf das Kopplungsstück auftreten, erstrecken sich der Vorsprung und die Ausnehmung vorzugsweise im Wesentlichen in axialer Richtung des Eingriffselements.

Um die medizinische Vorrichtung in relativ kleine Zuführsysteme, insbesondere in einen 0,017"-Katheter (0.432 mm) mit einem Innenlumen von 0,420 mm zuführen zu können, wird die Gitterstruktur auf einen relativ kleinen Querschnittdurchmesser komprimieren. Um dies zu bewirken, werden die beiden Crimphülsen, mit denen die formschlüssige Verbindung gesichert wird, axial hintereinander bzw. distal und/oder proximal zur Fügestelle angeordnet, wofür am Kopplungsstück vorzugsweise mehrere axial benachbarte Abschnitte vorgesehen sind, die jeweils unterschiedliche Außendurchmesser aufweisen. Dabei weist der Vorsprung den maximalen Außendurchmesser auf.

Die Crimphülse umgreift nicht die Fügestelle bzw. die formschlüssige Verbindung direkt, sondern ist entweder distal und/oder proximal dazu angeordnet. Hierfür ist vorzugsweise ein erster Abschnitt unmittelbar distal und ein zweiter Abschnitt unmittelbar proximal zum Vorsprung angeordnet. Der zweite Abschnitt kann einen größeren Außendurchmesser als der erste Abschnitt aufweisen.

In einer weiteren bevorzugten Ausführungsform ist ein dritter Abschnitt unmittelbar distal zum ersten Abschnitt angeordnet. Der dritte Abschnitt kann einen größeren Außendurchmesser als der erste Abschnitt aufweisen, wobei die Breite der beiden Abschnitte gleich ist. Dabei kann der dritte Abschnitt eine Erhöhung aufweisen, sodass durch den dritten Abschnitt ein Widerlager für die Crimpung gebildet wird, auf dem die Crimphülse aufliegt. Der dritte Abschnitt kann den gleichen Außendurchmesser wie der erste Abschnitt aufweisen.

In einer weiteren bevorzugten Ausführungsform ist ein vierter Abschnitt unmittelbar proximal zum zweiten Abschnitt angeordnet. Der vierte Abschnitt kann einen größeren Außendurchmesser als der zweite Abschnitt aufweisen, wobei die Breite der beiden Abschnitte gleich ist. Dabei kann der vierte Abschnitt eine Erhöhung aufweisen, sodass durch den vierten Abschnitt ein Widerlager für die Crimpung gebildet wird, auf dem die Crimphülse aufliegt. Der vierte Abschnitt kann den gleichen Außendurchmesser wie der zweite Abschnitt aufweisen.

In einer weiteren bevorzugten Ausführungsform ist ein fünfter Abschnitt unmittelbar proximal zum vierten Abschnitt angeordnet, wobei der fünfte Abschnitt einen größeren Außendurchmesser als der vierte Abschnitt aufweist. Das hat den Vorteil, dass die Crimphülse zusätzlich gegen axiales Verrutschen gesichert wird. Dabei erfolgt die axiale Ausrichtung des Kopplungsstücks durch das Einrasten des Vorsprungs in die Ausnehmung.

Vorzugsweise weist der fünfte Abschnitt eine Verjüngung auf, die sich in proximaler Richtung erstreckt. Dies erleichtert das Einführen und Zurückziehen der medizinischen Vorrichtung in den Katheter bzw. vermeidet ein Hängenbleiben.

In einer bevorzugten Ausführungsform ist wenigstens eine erste Crimphülse vorgesehen ist, die distal zum Vorsprung angeordnet ist und wenigstens einen Teilbereich des ersten Abschnitts und im Wesentlichen den gesamten Bereich des dritten Abschnitts umgreift. Die erste Crimphülse erfüllt eine Doppelfunktion. Einerseits stellt sie die Sicherung bzw. distale Unterstützung zur formschlüssigen Verbindung dar. Andererseits ermöglicht die erste Crimphülse zusätzlich das Fixieren eines Drahtelements am distalen Ende der Gitterstruktur. Des Weiteren stellt die erste Crimphülse ein röntgensichtbares Markerelement dar.

In einer weiteren bevorzugten Ausführungsform ist wenigstens eine zweite Crimphülse vorgesehen, die proximal zum Vorsprung angeordnet ist und wenigstens einen Teilbereich des zweiten Abschnitts und im Wesentlichen den gesamten Bereich des vierten Abschnitts umgreift. Durch die zweite Crimphülse wird die formschlüssige Verbindung zusätzlich in proximaler Richtung unterstützt, sodass eine noch stärkere Verbindungsstelle sichergestellt ist. Des Weiteren stellt die zweite Crimphülse ein röntgensichtbares Markerelement dar.

In einer bevorzugten Ausführungsform bilden die erste Crimphülse, die zweite Crimphülse, das Eingriffselement sowie das Kopplungsstück im zusammengebauten Zustand eine Reihenanordnung, wobei die Reihenanordnung vorzugsweise mit einem 0,017"-Katheter (0.432 mm) mit einem Innenlumen von 0,420 mm passgenau kompatibel ist. Der Vorteil der Reihenanordnung besteht darin, dass die beiden Crimphülsen flexibel angeordnet werden können. So können die beiden Crimphülsen beispielsweise axial hintereinander, insbesondere distal und/oder proximal zur Fügestelle angeordnet werden anstatt direkt an der Fügestelle selbst. Dies hat wiederum den Vorteil, dass die Fügestelle kleiner dimensioniert werden kann und dadurch weniger Bauraum einnimmt. Somit kann sich die Gitterstruktur auf einen relativ kleinen Querschnittdurchmesser komprimieren, sodass die medizinische Vorrichtung in relativ kleine Zuführsysteme zuführbar und insbesondere mit einem 0,017"-Katheter (0.432 mm) mit einem Innenlumen von 0,420 mm kompatibel ist.

In einer weiteren bevorzugten Ausführungsform weist das Eingriffselement vorzugsweise eine größere Breite als der Stegfortsatz auf. Einerseits ist dadurch die maximale Breite der Reihenanordnung festgelegt, die maßgebend für die Zuführbarkeit der medizinischen Vorrichtung in kleinere Zuführsysteme, insbesondere in einen 0,017"-Katheter (0.432 mm) mit einem Innenlumen von 0,420 mm ist.

Andererseits ist dadurch eine größere Ausbildung der Ausnehmung möglich, da um die Ausnehmung herum genügend Material vorhanden ist. Mit anderen Worten weist das Eingriffselement eine ausreichende Wandstärke auf, um höhere Zug-, Druck-, Biege- und Torsionskräfte übertragen zu können. Je größer die Ausnehmung bzw. der aufgrund der entsprechenden Negativform ausgebildete Vorsprung, desto stärker die formschlüssige Verbindung. Der Querschnitt der Ausnehmung und der Querschnitt des Stegfortsatzes sind gleich groß, sodass der verfügbare Bauraum optimal ausgenutzt wird. So ist über die Wirkfläche zwischen dem Vorsprung und der Ausnehmung eine gute Kraftübertragung möglich.

Vorzugsweise ist der Querschnitt des Eingriffselements trapezförmig ausgebildet, wobei die breitere Seite des Trapezes auf der Innenseite des Eingriffselements angeordnet ist. Das hat den Vorteil, dass das Eingriffselement weniger Bauraum einnimmt und dadurch gerade noch spielfrei durch die Crimphülsen hindurch passt.

Um eine optimale Ausnutzung des Querschnitts und die Spielfreiheit der Passung bei auftretender Torsion zu ermöglichen, weist das Eingriffselement im Bereich der Ausnehmung vorzugsweise zwei parallele Seiten auf, die sich in axialer Richtung des Eingriffselements erstrecken.

Um eine Fixierung eines Drahtelements mit einem proximalen Ende der Gitterstruktur zu ermöglichen, ist in einer bevorzugten Ausführungsform wenigstens ein röntgensichtbares Drahtelement vorgesehen, das entlang wenigstens eines Steges geführt ist. Das Drahtelement weist ein Drahtende auf, das durch die erste Crimphülse hindurchgeführt ist. Dabei ist das Drahtende durch die erste Crimphülse mit dem Stegfortsatz verbunden. Die erste Crimphülse kann sowohl ein Drahtende des Drahtelements mit der Gitterstruktur, als auch zwei Enden desselben Drahtelements oder zwei Drahtenden von verschiedenen Drahtelementen mit der Gitterstruktur verbinden. Es ist möglich, dass insgesamt vier Drahtenden fixiert werden. Dabei werden die Drahtenden sowohl durch die erste Crimphülse als auch durch die zweite Crimphülse geführt und sind mittig in den Eingriffselementen angeordnet. Die Verwendung einer Crimphülse zur Fixierung des Drahtelements an der Gitterstruktur, vorzugsweise an einem distalen Endabschnitt der Gitterstruktur, ist besonders einfach und kostengünstig möglich.

Um eine Gefäßverletzung während der Behandlung zu vermeiden, sind das Drahtende, die erste Crimphülse und die zweite Crimphülse mit dem Kopplungsstück klebeverbunden. Dabei kann die Verklebung als atraumatische Verklebung vorgesehen sein, die Kantenverrundungen aufweist und dadurch gewebeschonend ist.

Im Allgemeinen kann die erfindungsgemäß ausgebildete medizinische Vorrichtung ein Implantat, beispielsweise einen Stent, oder ein temporär einsetzbares Instrument, wie das zuvor beschriebene Thrombektomie-Device, bilden.

Ein weiterer nebengeordneter Aspekt der Erfindung betrifft ein Verfahren zur Herstellung einer zuvor beschriebenen medizinischen Vorrichtung, wobei ein Vorsprung, insbesondere ein Queranker durch eine Lasermaterialbearbeitung, Drahterodieren oder Schleifen an einem distalen Kopplungsstück eines Transportdrahtes hergestellt wird. Ferner wird eine Ausnehmung in einem Eingriffselement eines Stegfortsatzes durch ein Laserschneidverfahren hergestellt, wobei sich die Ausnehmung im Wesentlichen in axialer Richtung des Eingriffselements erstreckt. Die Ausnehmung kann durch ein 2-Achs-Laserschneidverfahren oder 4-Achs-Laserschneidverfahren hergestellt werden. Das Eingriffselement wird mit dem Kopplungsstück formschlüssig verbunden, wobei der Vorsprung in wenigstens eine Ausnehmung des Eingriffselements eingreift. Es wird wenigstens eine erste Crimphülse distal zum Vorsprung angeordnet, die wenigstens einen Teilbereich des ersten Abschnitts und im Wesentlichen den gesamten Bereich des dritten Abschnitts des Kopplungsstücks umgreift und wenigstens eine zweite Crimphülse proximal zum Vorsprung, die wenigstens einen Teilbereich des zweiten Abschnitts und im Wesentlichen den gesamten Bereich des vierten Abschnitts des Kopplungsstücks umgreift. Durch die erste Crimphülse, die zweite Crimphülse, das Eingriffselement sowie das Kopplungsstück wird im zusammengebauten Zustand eine Reihenanordnung gebildet, sodass die medizinische Vorrichtung in relativ kleine Zuführsysteme zuführbar ist.

Die Erfindung wird nachstehend anhand eines Ausführungsbeispiels unter Bezug auf die beigefügten schematischen Figuren mit weiteren Einzelheiten näher beschrieben.

In diesen zeigen
- Fig. 1: eine medizinische Vorrichtung nach einem erfindungsgemäßen Ausführungsbeispiel, wobei ein distales Kopplungsstück eines Transportdrahtes dargestellt ist;
- Fig. 2: eine medizinische Vorrichtung nach einem erfindungsgemäßen Ausführungsbeispiel, wobei ein proximales Ende der Gitterstruktur dargestellt ist;
- Fig. 3: eine medizinische Vorrichtung nach einem erfindungsgemäßen Ausführungsbeispiel, wobei eine formschlüssige Verbindung zwischen proximalen Ende der Gitterstruktur und dem distalen Ende des Transportdrahtes dargestellt ist (Montagezwischenzustand);
- Fig. 4: eine medizinische Vorrichtung nach einem erfindungsgemäßen Ausführungsbeispiel, wobei eine erste Crimphülse distal und eine zweite Crimphülse proximal zur formschlüssigen Verbindung angeordnet ist (Montagezwischenzustand);
- Fig. 5: eine medizinische Vorrichtung nach einem erfindungsgemäßen Ausführungsbeispiel, wobei die formschlüssige Verbindung durch Crimpen der beiden Crimphülsen gesichert ist (Montagezwischenzustand);
- Fig. 6: eine medizinische Vorrichtung nach einem erfindungsgemäßen Ausführungsbeispiel, wobei die formschlüssige Verbindung eine Verklebung aufweist (finaler Montagezustand);
- Fig. 7: eine medizinische Vorrichtung nach einem erfindungsgemäßen Ausführungsbeispiel, wobei die freien Drahtenden eines Drahtelements durch eine Crimphülse fixiert sind;
- Fig. 8: eine medizinische Vorrichtung nach einem erfindungsgemäßen Ausführungsbeispiel, wobei die formschlüssige Verbindung nur durch eine erste distale Crimphülse gesichert ist;
- Fig. 9: die medizinische Vorrichtung nach einem erfindungsgemäßen Ausführungsbeispiel, wobei der Querschnitt der Eingriffselemente dargestellt ist;
- Fig. 10: die medizinische Vorrichtung nach einem erfindungsgemäßen Ausführungsbeispiel, wobei die Gitterstruktur in einem flach ausgebreiteten Zustand dargestellt ist; und
- Fig. 11: die medizinische Vorrichtung gemäß Fig. 10 in einer Seitenansicht.

Im Allgemeinen weist die medizinische Vorrichtung eine Gitterstruktur auf, die aus miteinander verbundenen Stegen gebildet ist. Die Stege sind an Stegverbindern miteinander gekoppelt, wobei die Stegverbinder jeweils vier Stege miteinander verbinden können. Vorzugsweise ist die Gitterstruktur zumindest abschnittsweise rotationssymmetrisch geformt ausgebildet.

An dieser Stelle wird darauf hingewiesen, dass die Gitterstruktur röhrchenförmig ausgebildet ist. Die Röhrchenform ergibt sich daraus, dass die Gitterstruktur aus einem Rohrmaterial geschnitten ist, insbesondere lasergeschnitten ist. Andere Geometrien der Gitterstruktur sind möglich. Neben dem Laserschneiden sind andere Herstellungsverfahren möglich.

Die nachfolgend näher beschriebenen erfindungsgemäßen Ausführungsbeispiele zeigen jeweils medizinische Vorrichtungen, die zum intravaskulären Einsatz geeignet sind. Andere Arten von intravaskulär einsetzbaren medizinischen Vorrichtungen sind ebenfalls denkbar.

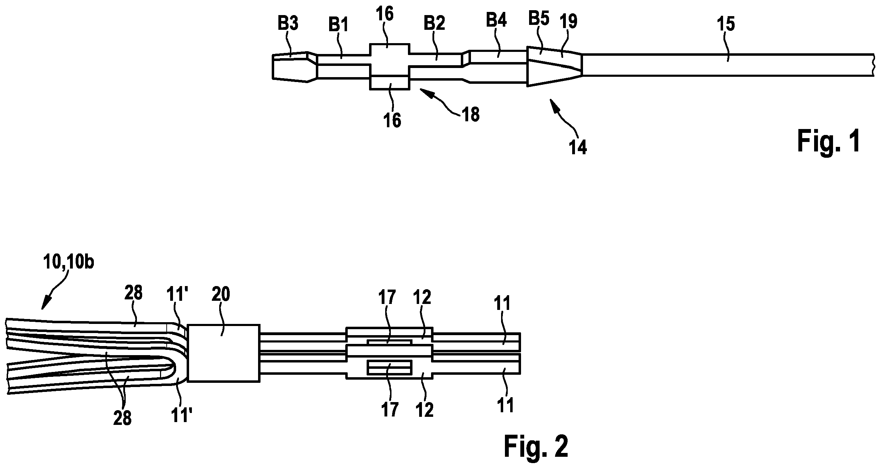

Fig. 1 zeigt das distale Ende eines Transportdrahts 15, an dem sich ein Kopplungsstück 14 befindet. Das Kopplungsstück 14 hat die Funktion, das proximale Ende der Gitterstruktur 10 fest mit dem Transportdraht 15 zu verbinden. Die Verbindung erfolgt dabei formschlüssig. Durch den Formschluss ist eine ausreichend starke Verbindung sichergestellt, die auftretende Zug-, Druck-, Biege- und Torsionskräfte aufnehmen bzw. übertragen kann. So kann die Gitterstruktur 10 für die Behandlung aus dem Katheter entlassen und nach der Thrombektomie wieder eingezogen werden.

Um die formschlüssige Verbindung zu ermöglichen, weist das Kopplungsstück 14 zwei Vorsprünge 16 auf, die sich im Wesentlichen quer zur Längsachse des Transportdrahts 15 erstrecken. Die Vorsprünge 16 sind als Queranker 18, insbesondere als T-Anker ausgebildet, sodass dadurch eine symmetrische Krafteinleitung möglich ist. Mit anderen Worten erstrecken sich die Vorsprünge 16 radial nach außen. Die Vorsprünge 16 können nahezu jede seitlich bzw. radial ausgeprägte Form aufweisen. Beispielweise können die Vorsprünge 16 eine runde, zickzack-förmige oder polygonale Außenkontur aufweisen, die sich in Umfangsrichtung und/oder Längsrichtung des Kopplungsstücks 14 erstreckt.

Einerseits sind die Vorsprünge 16 derart ausgebildet, dass sie gerade noch durch den Innendurchmesser einer Crimphülse zuführbar sind. Andererseits weisen sie einen ausreichenden Materialquerschnitt auf, um die auftretenden Zug-, Druck-, Biege- und Torsionskräfte übertragen zu können.

Es ist denkbar, dass das Kopplungsstück 14 mehrere gleichmäßig über den Umfang des Kopplungsstücks 14 verteilt angeordnete Vorsprünge 16 umfasst. So kann das Kopplungsstück 14 beispielsweise drei Vorsprünge 16 umfassen, die in Umfangsrichtung des Kopplungsstücks 14 angeordnet sind und jeweils einen Winkel von 120° zueinander aufweisen. Es können vier Vorsprünge 16 vorgesehen sein, die in Umfangsrichtung des Kopplungsstücks 14 angeordnet sind und jeweils einen Winkel von 90° zueinander aufweisen. Mit anderen Worten kann das Kopplungsstück 14 so viele Vorsprünge 16 wie möglich in Umfangsrichtung und/oder Längsrichtung des Kopplungsstücks 14 aufweisen, um eine stärkere formschlüssige Verbindung zu ermöglichen. Grundsätzlich reicht jedoch ein Vorsprung 16 aus, um eine ausreichend starke formschlüssige Verbindung sicherzustellen.

Das Kopplungsstück 14 ist im wesentlichen zylinderförmig ausgebildet und wird durch eine Lasermaterialbearbeitung, Drahterodieren oder Schleifen hergestellt, sodass das Kopplungsstück 14 wenigstens einen Vorsprung 16 enthaltenen Abschnitt aufweist.

Konkret umfasst das Kopplungsstück 14 mehrere axial benachbarte Abschnitte B1 bis B5, die jeweils unterschiedliche Außendurchmesser aufweisen, wobei der Vorsprung 16 den maximalen Außendurchmesser aufweist. Der erste Abschnitt B1 des Kopplungsstücks 14 ist unmittelbar distal zum Vorsprung 16 angeordnet. Der zweite Abschnitt B2 ist unmittelbar proximal zum Vorsprung 16 angeordnet. Der zweite Abschnitt B2 weist einen größeren Außendurchmesser als der erste Abschnitt B1 auf, um mögliche Drahtenden eines Drahtelements, insbesondere eines DFT-Drahtelements, mehr Raum bei einer distalen Crimpung zu bieten. Dies ist jedoch nicht zwingend erforderlich. Der erste Abschnitt B1 und der zweite Abschnitt B2 können auch denselben Außendurchmesser aufweisen

Der dritte Abschnitt B3 ist unmittelbar distal zum ersten Abschnitt B1 angeordnet, wobei der dritte Abschnitt B3 einen größeren Außendurchmesser als der erste Abschnitt B1 aufweist. Dabei ist die Breite des dritten Abschnitts B3 und des ersten Abschnitts B1 gleich. Der dritte Abschnitt B3 weist eine Erhöhung auf, um ein Widerlager für die Crimpung zu bilden.

Der vierte Abschnitt B4 ist unmittelbar proximal zum zweiten Abschnitt B2 angeordnet, wobei der vierte Abschnitt B4 einen größeren Außendurchmesser als der zweite Abschnitt B2 aufweist. Dabei ist die Breite des vierten Abschnitts B4 und des zweiten Abschnitts B2 gleich. Der vierte Abschnitt B4 weist eine Erhöhung auf, um ein Widerlager für die Crimpung zu bilden.

Der fünfte Abschnitt B5 ist unmittelbar proximal zum vierten Abschnitt B4 angeordnet, wobei der fünfte Abschnitt B5 einen größeren Außendurchmesser als der vierte Abschnitt B4 aufweist. Dadurch wird die Crimphülse zusätzlich gegen axiales Verrutschen gesichert. Der fünfte Abschnitt B5 weist des Weiteren eine Verjüngung 19 auf, die sich in proximaler Richtung erstreckt.

Fig. 2 zeigt die medizinische Vorrichtung nach einem erfindungsgemäßen Ausführungsbeispiel, wobei ein proximales Ende der Gitterstruktur dargestellt ist.

Konkret weist die Gitterstruktur 10 mehrere Stegfortsätze 11, insbesondere zwei Stegfortsätze 11 auf, die den in proximaler Richtung äußersten Bereich der Gitterstruktur 10 bilden.

Die Stegfortsätze 11 sind stiftartig ausgebildet und erstrecken sich ausgehend von einem Endverbinder 11' parallel zur Längsachse der Gitterstruktur 10. Der Endverbinder 11' verbindet zwei Stege 28 miteinander, die am proximalen Endabschnitt 10b der Gitterstruktur 10 angeordnet sind.

Jeweils ein Stegfortsatz 11 umfasst jeweils ein Eingriffselement 12, das eine Ausnehmung 17 aufweist. Dabei weisen die Ausnehmungen 17 im Wesentlichen dieselben Formen wie die Vorsprünge 16 auf. Somit kann jeweils ein Vorsprung 16 in jeweils eine Ausnehmung 17 eingreifen, sodass eine formschlüssige Verbindung geschaffen wird, die ausreichend stark ist, um die auftretenden Zug-, Druck, Biege- und Torsionskräfte zu übertragen.

Die Ausnehmungen 17 weisen eine geschlossene Form auf. Die Ausnehmungen 17 erstrecken sich im Wesentlichen in axialer Richtung der Eingriffselemente 12. Die Ausnehmungen 17 entsprechen der Kontur der Eingriffselemente 12. Es ist möglich, dass die Ausnehmungen 17 eine gekrümmte, insbesondere runde, zickzack-förmige oder polygonale Form aufweisen. Es ist vorgesehen, dass die Ausnehmungen 17 jeweils wenigstens zwei gegenüberliegende Seiten umfassen. Konkret weisen die Ausnehmungen 17 jeweils eine rechteckige geschlossene Form auf, die sich in axialer Richtung bezogen auf das Eingriffselement 12 erstrecken.

Die Ausnehmungen 17 werden durch ein Laserschneidverfahren hergestellt. Die Ausnehmungen 17 können durch ein konventionelles 2-Achs-Laserschneidverfahren oder 4-Achs-Laserschneidverfahren hergestellt werden. So können jeweils die beiden in axialer Richtung verlaufenden Seiten der Ausnehmungen 17 exakt parallel anstatt konisch zueinander geschnitten werden, sodass die Ausnehmungen 17 die entsprechenden Negativformen der Vorsprünge 16 bilden. Dies ermöglicht, dass die Vorsprünge 16 passgenau in die Ausnehmungen 17 angeordnet werden können.

Bei Herstellung der Ausnehmungen 17 durch ein konventionelles 2-Achs-Laserschneidverfahren können bereits ca. 70 % der auftretenden Kräfte übertragen werden.

Zur Unterstützung und Sicherung der formschlüssigen Verbindung ist eine erste Crimphülse 20 vorgesehen, die die Stegfortsätze 11 umgreift und distal zu den Eingriffselementen 12 angeordnet ist. Auf die erste Crimphülse 20 wird nachfolgend näher eingegangen.

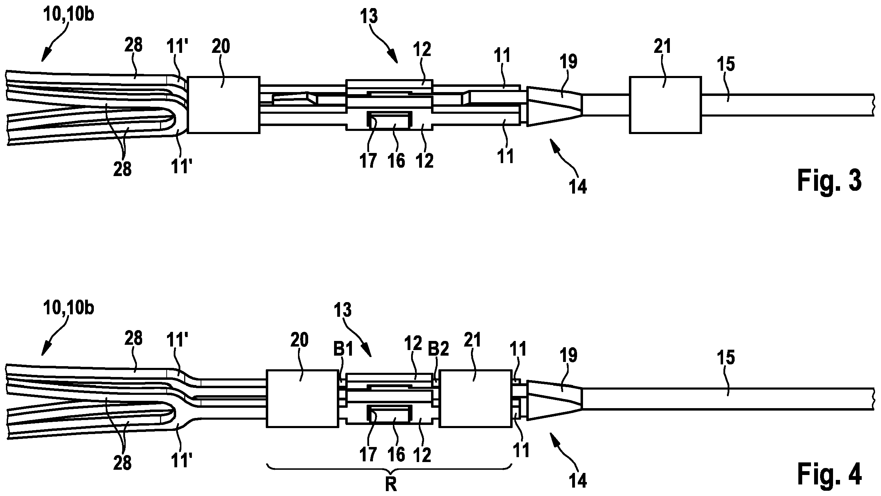

Fig. 3 zeigt einen Montagezwischenschritt, bei der die formschlüssige Verbindung 13 zwischen den Eingriffselementen 12 und dem Kopplungsstück 14 dargestellt ist.

Die formschlüssige Verbindung 13 ermöglicht eine ausreichend starke Verbindung, um die auftretenden Zug-, Druck-, Biege- und Torsionskräfte übertragen zu können. Da diese eine lösbare Verbindung darstellt, wird diese zusätzlich gesichert. Ohne eine zusätzliche Sicherung könnte sich die formschlüssige Verbindung 13 lösen, da die Stegfortsätze 11 freie Enden aufweisen.

Um das Aufspreizen der Stegfortsätze 11 zu verhindern bzw. die formschlüssige Verbindung 13 zu sichern, ist wenigstens eine erste Crimphülse 20 vorgesehen. Dabei wird die erste Crimphülse 20 zuerst über die formschlüssige Verbindung 13 aufgeschoben und soweit wie möglich in distaler Richtung angeordnet, damit die Stegfortsätze 11 ausreichend aufgespreizt und die Vorsprünge 16 dadurch passgenau in die Ausnehmungen 17 angeordnet werden können.

Danach werden das Eingriffselement 12 und das Kopplungselement 14 relativ zu einander positioniert, um diese miteinander formschlüssig zu verbinden. Die formschlüssige Verbindung 13 entsteht, indem die Vorsprünge 16 in die Ausnehmungen 17 eingreifen. Vorzugsweise greifen die Vorsprünge 16 passgenau in die Ausnehmungen 17 ein. Die axiale Ausrichtung wird durch das Einrasten der Vorsprünge 16 in die Ausnehmungen 17 erreicht.

Durch die formschlüssige Verbindung 13 können Kräfte, insbesondere Zug- und Druckkräfte in axialer Richtung bezogen auf das Kopplungsstück sowie Biege- und Torsionskräfte übertragen werden, die während der Behandlung, insbesondere wenn die medizinische Vorrichtung nach einem Positionierungsversuch wieder in ein Zuführsystem, beispielsweise einen Katheter, zurückgezogen werden soll, auftreten.

Anschließend wird eine zweite Crimphülse 21 über das Kopplungsstück 14 aufgeschoben, wobei das Kopplungsstück 14 eine Verjüngung 19 aufweist, um das Aufschieben der zweiten Crimphülse 21 zu erleichtern.

Die Crimphülsen 20, 21 können vorzugsweise röntgensichtbar sein bzw. ein röntgensichtbares Material aufweisen. Die Crimphülsen 20, 21 können selbst röntgensichtbare Markerelemente darstellen. Damit wird ermöglicht, dass das proximale Ende der Gitterstruktur 10 unter einer Röntgenkontrolle besonders gut sichtbar ist. Auf diese Weise wird die Positionierung der Gitterstruktur 10 beispielsweise in einem Blutgefäß erleichtert.

Fig. 4 zeigt den Montagezwischenschritt, bei der eine erste Crimphülse distal und eine zweite Crimphülse proximal zur formschlüssigen Verbindung angeordnet ist.

Die erste Crimphülse 20, die zweite Crimphülse 21 und die Eingriffselemente 12 sind axial hintereinander angeordnet, sodass sie zusammen mit dem Kopplungsstück 14 eine Reihenanordnung R bilden. Dabei umgreift die erste Crimphülse 20 einen distalen Bereich der Stegfortsätze 11 sowie wenigstens einen Teilbereich des ersten Abschnitts B1 und im Wesentlichen den gesamten Bereich des dritten Abschnitts B3 des Kopplungselements 14, sodass die erste Crimphülse 20 eine distale Sicherung der formschlüssigen Verbindung 13 darstellt. Die zweite Crimphülse 21 umgreift einen proximalen Bereich der Stegfortsätze 11 sowie wenigstens einen Teilbereich des zweiten Abschnitts B2 und im Wesentlichen den gesamten Bereich des vierten Abschnitts B4 des Kopplungselements 14, sodass die zweite Crimphülse 21 eine proximale Sicherung der formschlüssigen Verbindung 13 darstellt. Mit anderen Worten hat die erste Crimphülse 20 die Aufgabe die formschlüssige Verbindung 13 distal und die zweite Crimphülse 21 die Aufgabe die formschlüssige Verbindung 13 proximal zu sichern. Der dritte Abschnitt B3 und der vierte Abschnitt B4 des Kopplungselements 14 weisen eine Erhöhung auf, um ein Widerlager für die Crimpung zu bilden.

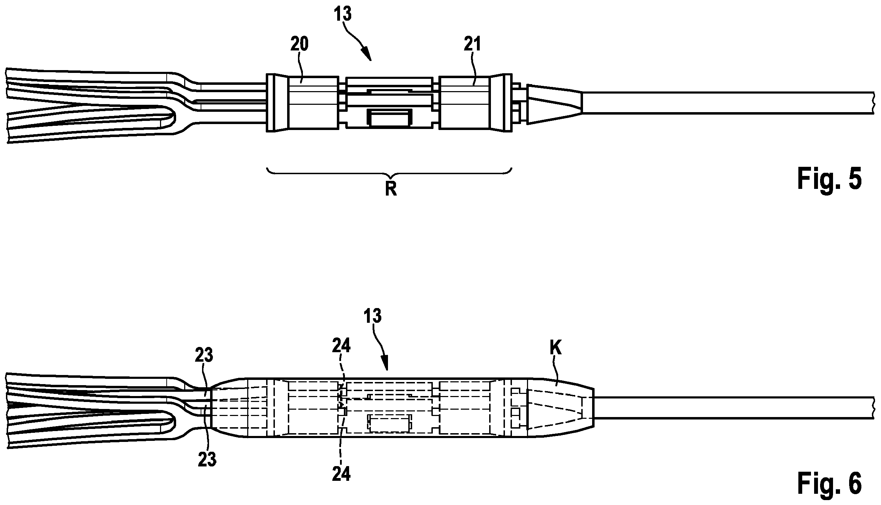

Fig. 5. zeigt den Zwischenmontagezustand, bei der die formschlüssige Verbindung durch Crimpen der beiden Crimphülsen gesichert ist.

Im Vergleich zum direkten Umgreifen der Fügestelle durch eine einzige Crimphülse bzw. das Fixieren der formschlüssigen Verbindung durch ein aufeinander Anordnen einer Crimphülse und der Fügestelle, hat die Reihenanordnung den Vorteil, dass die Crimphülsen axial hintereinander angeordnet werden können. Die Fixierung der formschlüssigen Verbindung erfolgt dabei flexibel, insbesondere distal und/oder proximal zur formschlüssigen Verbindung. Konkret fixiert die erste Crimphülse 20 die formschlüssige Verbindung 13 distal und die zweite Crimphülse 21 die formschlüssige Verbindung 13 proximal. Dadurch weisen die formschlüssige Verbindung 13 sowie die beiden Crimphülsen 20, 21 etwa den gleichen, insbesondere einen kleinen Außendurchmesser auf. Dadurch wird weniger Bauraum eingenommen und die Gitterstruktur 10 kann somit auf einen relativ kleinen Querschnittdurchmesser komprimiert werden, sodass die medizinische Vorrichtung in relativ kleine Zuführsysteme zuführbar und insbesondere mit einem 0,017"-Katheter (0.432 mm) mit einem Innenlumen von 0,420 mm kompatibel ist.

Fig. 6. zeigt den finalen Montagezustand der formschlüssigen Verbindung, die eine Verklebung K aufweist. Die Verklebung K ist als atraumatische Verklebung ausgebildet, d.h. die Verklebung K weist Kantenverrundungen auf und ist dadurch gewebeschonend. Somit lassen sich Gefäßverletzungen während der Behandlung vermeiden. Des Weiteren wird durch die Verklebung K das Einführen und Zurückziehen in den Katheter erleichtert, da die formschlüssige Verbindung dadurch besser gleiten kann und ein Hängenbleiben vermieden wird.

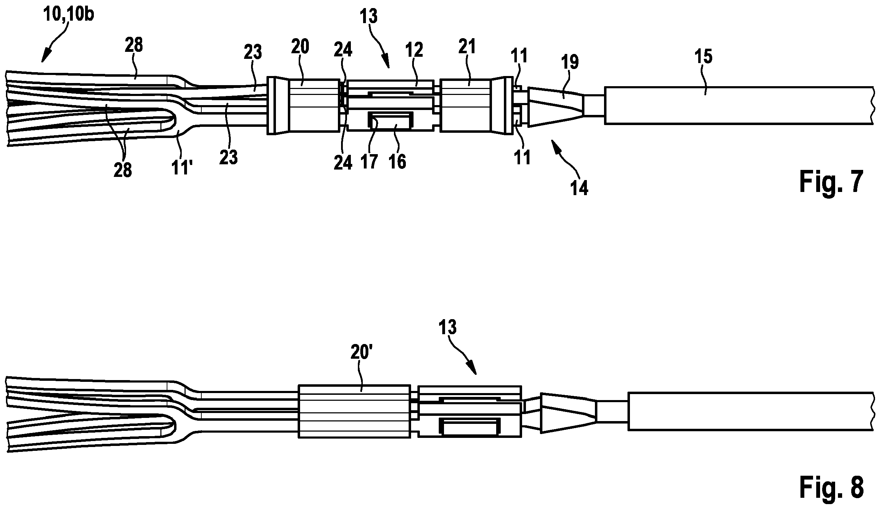

Fig. 7 zeigt einen Montagezustand der formschlüssigen Verbindung, bei der die freien Drahtenden 23 eines Drahtelements 24 durch die erste Crimphülse 20 hindurchgeführt sind. Dabei sind die freien Drahtenden 23 durch einen Zwischenraum geführt, der zwischen der ersten Crimphülse 20, dem dritten Abschnitt B3 und den Stegfortsätzen 11 der Gitterstruktur 10 ausgebildet ist.

Das Drahtelement bzw. die Drahtelemente 24 sind aus einem röntgensichtbaren Material gebildet. Beispielsweise kann es sich um ein Vollmaterial aus einem röntgensichtbaren Werkstoff handeln. Es ist auch möglich, ein Verbundmaterial, beispielsweise sogenannte DFT-Drahtelemente einzusetzen. Derartige Drahtelemente weisen einen Kerndraht aus einem röntgensichtbaren Material, beispielsweise Platin, Tantal oder Gold, und ein Mantel aus einem Formgedächtnismaterial auf.

Ein Beispiel für ein DFT-Drahtelement weist einen Platinkern von 45-55 µm Durchmesser und einem Nitinolmantel, dessen Außendurchmesser dem Kerndurchmesser plus ca. 20 µm entspricht. Denkbar wären auch andere Materialien und variierte Abmessungen oder nur ein röntgensichtbarer Draht ohne superelastischen Mantel.

Generell können alle für Implantate üblichen röntgensichtbaren Materialien verwendet werden.

Die erste Crimphülse 20 erfüllt hierbei eine Doppelfunktion. Einerseits stellt sie eine distale Sicherung der formschlüssigen Verbindung 13 sicher. Andererseits ermöglicht sie das zusätzliche Fixieren des Drahtelements 24 an einem proximalen Ende der Gitterstruktur 10. Dabei kann die erste Crimphülse 20 nicht nur die freien Drahtenden 23 eines einzigen Drahtelements 24 miteinander an der Gitterstruktur 10 fixieren, sondern auch Drahtenden 23 verschiedener Drahtelemente 24.

Es ist möglich, dass insgesamt vier Drahtenden 23 fixiert werden. Dabei werden die Drahtenden 23 sowohl durch die erste Crimphülse 20 als auch durch die zweite Crimphülse 21 geführt und sind mittig in den Eingriffselementen 12 angeordnet.

Fig. 8 zeigt die medizinische Vorrichtung nach einem erfindungsgemäßen Ausführungsbeispiel, wobei die formschlüssige Verbindung durch lediglich eine Crimphülse gesichert ist, die distal zur formschlüssigen Verbindung angeordnet ist.

Im Gegensatz zu den zuvor beschriebenen Crimphülsen 20, 21 ist die in Fig. 8 dargestellte Crimphülse 20' breiter bzw. länger in axialer Richtung ausgebildet. Der Einsatz einer derart breiten Crimphülse 20' hat den Vorteil, dass auf eine zusätzliche zweite Crimphülse 21 verzichtet werden kann, die erforderlich wäre, um die formschlüssige Verbindung 13 proximal zu sichern.

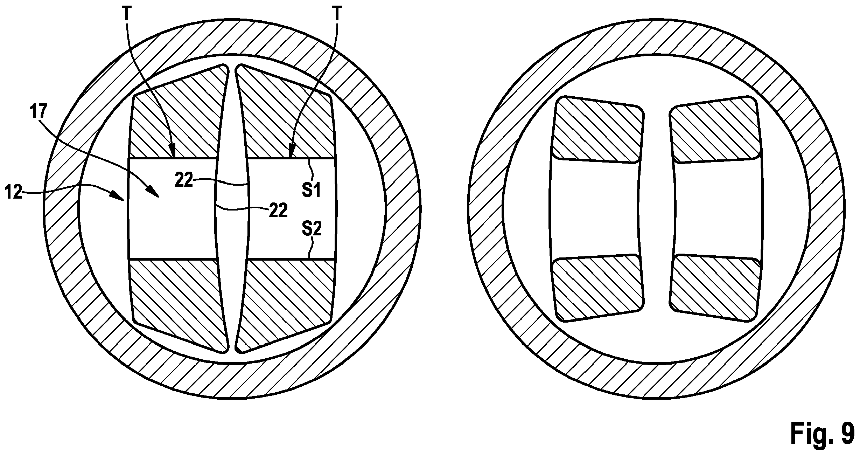

Fig. 9 zeigt die medizinische Vorrichtung nach einem erfindungsgemäßen Ausführungsbeispiel, wobei der Querschnitt der Eingriffselemente dargestellt ist.

Die Eingriffselemente 12 weisen eine größere Breite als die Stegfortsätze 11 auf. Einerseits ist dadurch die maximale Breite der Fügestelle festgelegt. Andererseits ist dadurch eine größere Ausbildung der Ausnehmungen 17 möglich, da um die Ausnehmungen 17 herum genügend Material vorhanden ist. Die Eingriffselemente 12 weisen somit eine ausreichende Wandstärke auf, um die auftretenden Zug-, Druck-, Biege- und Torsionskräfte übertragen zu können.

Die beiden Stege ober- und unterhalb der Ausnehmungen 17 weisen zusammen optimalerweise denselben Querschnitt wie die Ausnehmungen 17 auf. Die Ausnehmungen 17 weisen optimalerweise denselben Querschnitt wie die Stegfortsätze 11 auf. Das optimale Verhältnis zwischen dem Querschnitt der Ausnehmungen 17 und dem Querschnitt der Stegfortsätze 11 beträgt 2:1.

Um die erste Crimphülse 20 gerade noch über die Eingriffselemente 12 aufschieben zu können, sind die Querschnitte der Eingriffselemente 12 trapezförmig ausgebildet. Dabei sind die breiteren Seiten 22 der Trapeze T auf der Innenseite der Eingriffselemente 12 angeordnet. Dies lässt sich gut in Fig. 9 erkennen.

Die Trapeze T sind durch ein "Off-Center-Schnittverfahren" hergestellt und weisen im Bereich der Ausnehmungen 17 zwei parallele Seiten S1 und S2 auf, die für die Spielfreiheit bei auftretenden Torsionskräfte wichtig sind. Die parallelen Seiten S1, S2 erstrecken sich über die gesamten axialen Längen der Ausnehmungen 17. Durch das "Off-Center-Schnittverfahren" wird gegenüber dem reinen radialen Schnittverfahren ein Materialquerschnittgewinn von ca. 30 % bewirkt.

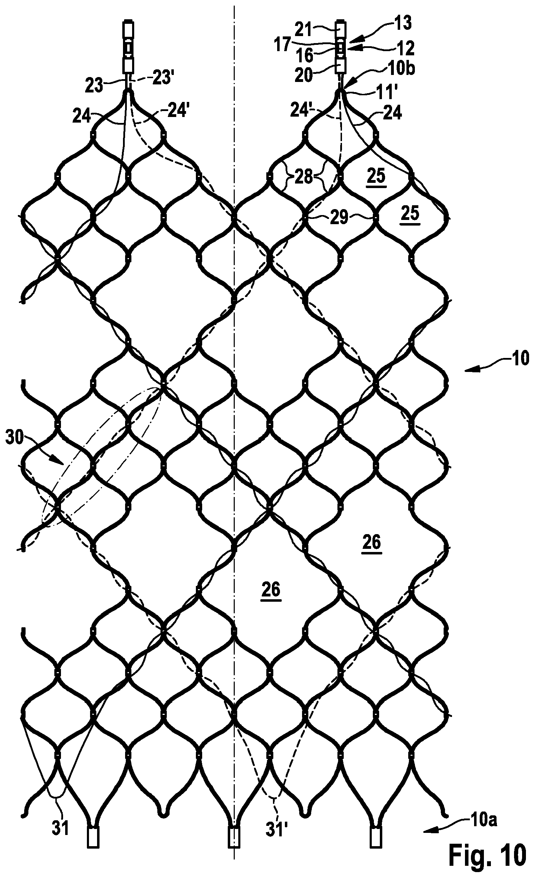

Fig. 10 zeigt die medizinische Vorrichtung nach einem erfindungsgemäßen Ausführungsbeispiel, wobei die Gitterstruktur 10 in einem flach ausgebreiteten Zustand dargestellt ist. Die Gitterstruktur 10 ist konkret als Stentkomponente ausgebildet, die zur schnellen Rekanalisation geeignet ist, wobei das proximale Ende der Stentkomponente mit einem distalen Kopplungsstück 14 eines nicht dargestellten Transportdrahtes 15 durch eine wie in Fig. 6 und Fig. 7 dargestellte formschlüssigen Verbindung 13 verbunden ist.

Die Gitterstruktur 10 bzw. Stentkomponente weist ein hybrides Zelldesign auf, d.h. die Gitterstruktur 10 umfasst einerseits geschlossene Zellen 25, die eine hervorragende Apposition an die Gefäßwand bewirken sowie eine sichere Aufnahme von Thromben ermöglichen, und andererseits größere Zellen 26. Die größeren Zellen 26 können in einigen Ausführungsformen Verankerungselemente aufweisen, die ein wirksames Festhalten der Thromben sowie eine sichere, atraumatische Entfernung ermöglichen. Derartige Verankerungselemente können durch jeweils zwei Zusatzstege der Gitterstruktur 10 gebildet sein, die V-förmig zu einer Spitze verbunden sind, die in die größere Zelle 26 hineinragt.

Grundsätzlich sind die Zellen 25, 26 der Gitterstruktur 10 jeweils aus mehreren, einstückig miteinander verbundenen Stegen 28 gebildet. Die Stege 28 sind an Stegverbindern 29 miteinander gekoppelt, wobei die Stegverbinder 29 jeweils konkret vier Stege 28 miteinander verbinden. Die an die größeren Zellen 26 angrenzenden Stegverbinder 29 verbinden allerdings nur jeweils drei Stege 28 miteinander.

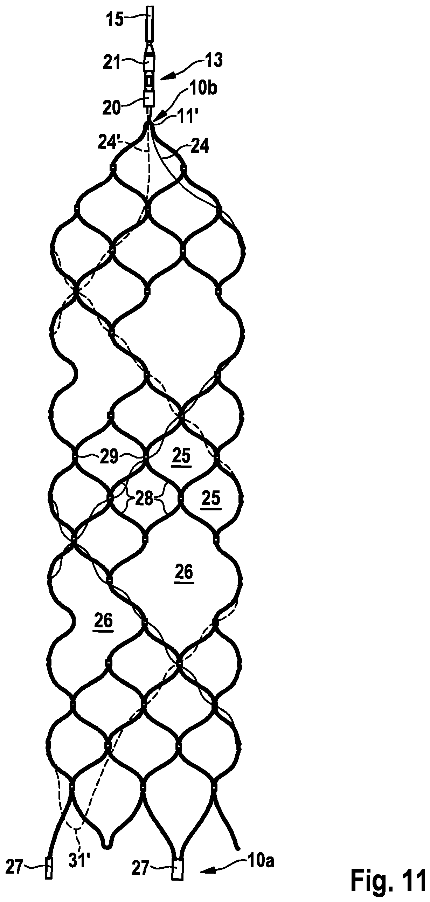

Aus Darstellungsgründen wurde in Fig. 10 auf eine entfaltete Ansicht zurückgegriffen, in der die Gitterstruktur 10 in Längsrichtung aufgeschnitten und flach ausgebreitet gezeigt ist. Es versteht sich, dass die Gitterstruktur 10 tatsächlich röhrchenförmig oder zumindest abschnittsweise röhrchenförmig ausgebildet ist. Dies ist in der Seitenansicht gemäß Fig. 11 erkennbar. Die Röhrchenform ergibt sich daraus, dass die Gitterstruktur 10 aus einem Rohrmaterial geschnitten, insbesondere lasergeschnitten, ist.

Die Gitterstruktur 10 weist mehrere stiftartig ausgebildete Stegfortsätze 11 auf, die den in proximaler Richtung äußersten Bereich, insbesondere den proximalen Endabschnitt 10b, der Gitterstruktur 10 bilden. In Fig. 10 sind konkret zwei Stegfortsätze 11 dargestellt, wobei jeweils ein Stegfortsatz 11 ein Eingriffselement 12 aufweist, sodass eine zuvor beschriebene formschlüssige Verbindung 13 mit einem distalen Kopplungsstück 14 eines Transportdrahtes 15 möglich ist. Das Eingriffselement 12 umfasst eine rechteckige Ausnehmung 17, die formschlüssig mit einem Vorsprung 16 des Kopplungsstücks 14 zusammenwirkt.

Die formschlüssige Verbindung 13 zwischen der Gitterstruktur 10 der Stentkomponente und dem Transportdraht 15 wird konkret dadurch ermöglicht, dass der in Fig. 1 dargestellte Vorsprung 16 des Kopplungsstücks 14 in die in Fig. 2 dargestellte, vorzugsweise rechteckige, Ausnehmung 17 des Eingriffselements 12 eingreift, sodass eine ausreichend starke Verbindung zwischen der Stentkomponente und dem Transportdraht 15 geschaffen ist.

Die medizinische Vorrichtung gemäß Fig. 10 umfasst zwei Drahtelemente 24, 24', sodass insgesamt vier Drahtenden 23, 23' durch die in Fig. 10 nicht dargestellte erste Crimphülse 20 fixiert werden. Die erste Crimphülse 20 entspricht der ersten Crimphülse 20, wie sie in den Fig. 6 und 7 gezeigt ist.

Es ist möglich, dass die Drahtenden 23, 23' bzw. sowohl durch die erste Crimphülse 20 als auch durch die zweite Crimphülse 21 geführt und mittig in den Eingriffselementen 12 angeordnet und fixiert sind. Es ist möglich, dass mehr als zwei Drahtelemente 24, 24' vorgesehen sind, beispielsweise insgesamt drei oder vier Drahtelemente 24, 24', die musterartig auf dem Umfang verteilt angeordnet sind.

Zur Befestigung sind die Drahtelemente 24, 24' bereichsweise in die Gitterstruktur 10 eingeflochten und/oder umwickeln einzelne Stege 28 der Gitterstruktur 10. Mehrere Drahtelemente können unabhängig voneinander mit der Gitterstruktur 10 verflochten sein und/oder die einzelnen Stege 28 der Gitterstruktur 10 umwickeln.

Die Drahtelemente 24, 24' verlaufen im Wesentlichen parallel bzw. seitlich entlang der Stege 28. Im Allgemeinen erstrecken sich die Drahtelemente 24, 24' vorzugsweise entlang einer Stegreihe 30, die durch mehrere hintereinander angeordnete Stege 28 gebildet ist. Die Stegreihe 30 verläuft vorzugsweise helixförmig um die Längsachse der Gitterstruktur 10.

Zusätzlich können die Drahtelemente 24, 24' um die Stege 28 der Stegreihe 30 gewickelt sein. Die Drahtelemente 24, 24' erreichen eine volle Windung, insbesondere eine Windung um 360°, nach einem Steg 28 bzw. nach einer Steglänge, wobei die Länge eines Stegs 28 dem entlang des Stegs 28 zu ermittelnden Abstand zweier Kreuzungspunkte von Stegen 28 entspricht.

Die Drahtelemente 24, 24' bilden Schlaufen 31, 31', die im Bereich des distalen Endabschnitts 10a der Gitterstruktur 10 angeordnet sind. Der distale Endabschnitt 10a bildet das axiale Ende der röhrchenförmigen Gitterstruktur 10, das beim Entlassen der Vorrichtung aus einem Katheter zuerst austritt.

Im Bereich der Schlaufen 31, 31' ändern die Drahtelemente 24, 24' ihre Richtung und zwar so, dass die Drahtelemente 24, 24' aus der Gitterstruktur 10 heraus- und wieder hereingeführt werden. Die Schlaufen 31, 31' weisen jeweils einen ersten Schenkel und einen zweiten Schenkel auf, die sich unter einem Winkel zueinander erstrecken.

Fig. 11 zeigt die medizinische Vorrichtung gemäß Fig. 10 nochmals in einer Seitenansicht. Es ist gut erkennbar, dass die Stentkomponente bzw. Gitterstruktur 10 mit dem Transportdraht 15 über das Kopplungsstück 14 des Transportdrahts 15 und das Eingriffselement 12 der Gitterstruktur 10 fest, insbesondere unter Bildung der Formschlussverbindung 13, verbunden ist. Die insgesamt vier Drahtenden 23, 23', von welchen in der Seitenansicht lediglich zwei erkennbar sind, sind mit der ersten Crimphülse 20 am Kopplungsstück 14 und/oder an den Stegfortsätzen 11 fixiert.

Am einem distalen Endabschnitt 10a der Gitterstruktur 10 sind Röntgenmarkerhülsen 27 vorgesehen. Dazu gehen von den Endverbindern 11' der Gitterstruktur 10 am distalen Endabschnitt 10a jeweils Stegstifte aus, die sich parallel zur Längsrichtung der Gitterstruktur 10 erstrecken. Auf die Stegstifte sind die Röntgenmarkerhülsen 27 aufgecrimpt. Die Röntgenmarkerhülsen 27 können ein röntgensichtbares Material, insbesondere Gold, Tantal, Platin oder Legierungen hiervon, aufweisen.

Auch die Crimphülsen 20, 21 können ein röntgensichtbares Material aufweisen, beispielsweise Gold, Tantal, Platin oder Legierungen hiervon. Insofern können die Crimphüsen 20, 21 am proximalen Endabschnitt 10b der Gitterstruktur 10 bzw. am distalen Ende des Transportdrahts 15 also ebenso wie die Röntgenmarkerhülsen 27 am distalen Endabschnitt 10a der Gitterstruktur 10 eine Röntgensichtbarkeitsfunktion erfüllen.

### Bezuaszeichenliste

- 10: Gitterstruktur
- 10a: distaler Endabschnitt
- 10b: proximaler Endabschnitt
- 11: Stegfortsatz
- 11': Endverbinder
- 12: Eingriffselement
- 13: formschlüssige Verbindung
- 14: Kopplungsstück
- 15: Transportdraht
- 16: Vorsprung
- 17: Ausnehmung
- 18: Queranker
- 19: Verjüngung
- 20: erste Crimphülse
- 20': breite Crimphülse
- 21: zweite Crimphülse
- 22: breitere Seite
- 23: Drahtende
- 23': Drahtende
- 24: Drahtelement
- 24': Drahtelement
- 25: Zelle
- 26: Zelle
- 27: Röntgenmarkerhülse
- 28: Steg
- 29: Stegverbinder
- 30: Stegreihe
- 31: Schlaufe
- 31': Schlaufe
- B1: erste Abschnitt
- B2: zweiter Abschnitt
- B3: dritter Abschnitt
- B4: vierter Abschnitt
- B5: fünfter Abschnitt
- K: Verklebung
- R: Reihenanordnung
- S1: erste parallele Seite
- S2: zweite parallele Seite
- T: Trapez

## Patentansprüche

1. Medizinische Vorrichtung, insbesondere Thrombektomie-Device, zur intravaskulären Behandlung mit einer zumindest abschnittsweise rohrförmigen Gitterstruktur (10) aus einstückig miteinander verbundenen Stegen (28), wobei die Gitterstruktur (10) an einem proximalen Endabschnitt (10b) einen Stegfortsatz (11) aufweist, der ein Eingriffselement (12) umfasst, wobei das Eingriffselement (12) durch eine formschlüssige Verbindung (13) mit einem distalen Kopplungsstück (14) eines Transportdrahts (15) verbunden ist und das Kopplungsstück (14) wenigstens einen Vorsprung (16) und das Eingriffselement (12) wenigstens eine Ausnehmung (17) aufweisen, wobei der Vorsprung (16) in die Ausnehmung (17) des Eingriffselements (12) eingreift,
**dadurch gekennzeichnet, dass**
die Ausnehmung (17) geschlossen ausgebildet ist, wobei sich die Ausnehmung im Wesentlichen in axialer Richtung des Eingriffselements (12) erstreckt.

2. Medizinische Vorrichtung nach Anspruch 1
**dadurch gekennzeichnet, dass**
die Ausnehmung (17) im Wesentlichen der Kontur des Eingriffselements (12) entspricht.

3. Medizinische Vorrichtung nach Anspruch 1 oder 2
**dadurch gekennzeichnet, dass**
das Kopplungsstück (14) wenigstens zwei Vorsprünge (16) aufweist, die als Queranker (18) ausgebildet sind.

4. Medizinische Vorrichtung nach einem der vorhergehenden Ansprüche
**dadurch gekennzeichnet, dass**
der Queranker (18) als T-Anker ausgebildet ist.

5. Medizinische Vorrichtung nach einem der vorhergehenden Ansprüche
**dadurch gekennzeichnet, dass**
der Vorsprung (16) der Form der Ausnehmung (17) entspricht, insbesondere wobei sich der Vorsprung (16) im Wesentlichen in axialer Richtung des Kopplungsstücks (14) erstreckt.

6. Medizinische Vorrichtung nach einem der vorhergehenden Ansprüche
**dadurch gekennzeichnet, dass**
das Kopplungsstück (14) mehrere axial benachbarte Abschnitte (B1-B5) umfasst, die jeweils unterschiedliche Außendurchmesser aufweisen, wobei der Vorsprung (16) den maximalen Außendurchmesser aufweist, insbesondere wobei ein erster Abschnitt (B1) unmittelbar distal und ein zweiter Abschnitt (B2) unmittelbar proximal zum Vorsprung (16) angeordnet sind.

7. Medizinische Vorrichtung nach Anspruch 6
**dadurch gekennzeichnet, dass**
ein dritter Abschnitt (B3) unmittelbar distal zum ersten Abschnitt (B1) angeordnet ist und/oder dass ein vierter Abschnitt (B4) unmittelbar proximal zum zweiten Abschnitt (B2) angeordnet ist, insbesondere wobei ein fünfter Abschnitt (B5) unmittelbar proximal zum vierten Abschnitt (B4) angeordnet ist, wobei der fünfte Abschnitt (B5) einen größeren Außendurchmesser als der vierte Abschnitt (B4) aufweist, insbesondere wobei der fünfte Abschnitt (B5) eine Verjüngung (19) aufweist, die sich in proximaler Richtung erstreckt.

8. Medizinische Vorrichtung nach Anspruch 6 oder 7
**dadurch gekennzeichnet, dass**
wenigstens eine erste Crimphülse (20) vorgesehen ist, die distal zum Vorsprung (16) angeordnet ist und wenigstens einen Teilbereich des ersten Abschnitts (B1) und im Wesentlichen den gesamten Bereich des dritten Abschnitts (B3) umgreift.

9. Medizinische Vorrichtung nach einem der Ansprüche 6 bis 8
**dadurch gekennzeichnet, dass**
wenigstens eine zweite Crimphülse (21) vorgesehen ist, die proximal zum Vorsprung (16) angeordnet ist und wenigstens einen Teilbereich des zweiten Abschnitts (B2) und im Wesentlichen den gesamten Bereich des vierten Abschnitts (B4) umgreift.

10. Medizinische Vorrichtung nach einem der vorhergehenden Ansprüche
**dadurch gekennzeichnet, dass**
die erste Crimphülse (20), die zweite Crimphülse (21), das Eingriffselement (12) sowie das Kopplungsstück (14) im zusammengebauten Zustand eine Reihenanordnung (R) bilden, insbesondere wobei die Reihenanordnung (R) mit einem 0,017"-Kather mit einem Innenlumen von 0,420 mm passgenau kompatibel ist.

11. Medizinische Vorrichtung nach einem der vorhergehenden Ansprüche
**dadurch gekennzeichnet, dass**
das Eingriffselement (12) eine größere Breite als der Stegfortsatz (11) aufweist, insbesondere wobei der Querschnitt des Eingriffselements (12) trapezförmig ausgebildet ist, wobei die breitere Seite (22) des Trapezes (T) auf der Innenseite des Eingriffselements (12) angeordnet ist, insbesondere wobei das Eingriffselement (12) im Bereich der Ausnehmung (17) zwei parallele Seiten (S1, S2) aufweist, die sich in axialer Richtung des Eingriffselements (12) erstrecken.

12. Medizinische Vorrichtung nach einem der Ansprüche 8 bis 11
**dadurch gekennzeichnet, dass**
wenigstens ein röntgensichtbares Drahtelement (24) vorgesehen ist, das entlang wenigstens eines Steges geführt ist, wobei das Drahtelement (24) ein Drahtende (23) aufweist, das durch die erste Crimphülse (20) hindurchgeführt ist, insbesondere wobei das Drahtende (23), die erste Crimphülse (21) und die zweite Crimphülse (22) mit dem Kopplungsstück (14) klebeverbunden sind.

13. Verfahren zur Herstellung einer medizinischen Vorrichtung nach einem der vorhergehenden Ansprüche, wobei
- ein Vorsprung (16), insbesondere ein Queranker (18) durch eine Lasermaterialbearbeitung, Drahterodieren oder Schleifen an einem distalen Kopplungsstück (14) eines Transportdrahtes (15) hergestellt wird;
- eine Ausnehmung (17) in einem Eingriffselement (12) eines Stegfortsatzes (11) durch ein Laserschneidverfahren hergestellt wird, wobei sich die Ausnehmung (17) im Wesentlichen in axialer Richtung des Eingriffselements (12) erstreckt;
- das Eingriffselement (12) mit dem Kopplungsstück (14) formschlüssig verbunden wird, wobei der Vorsprung (16) in wenigstens eine Ausnehmung (17) des Eingriffselements (12) eingreift;
- wenigstens eine erste Crimphülse (20) distal zum Vorsprung (16) angeordnet wird, die wenigstens einen Teilbereich des ersten Abschnitts (B1) und im Wesentlichen den gesamten Bereich des dritten Abschnitts (B3) umgreift.

14. Verfahren nach Anspruch 13,
**dadurch gekennzeichnet, dass**
wenigstens eine zweite Crimphülse (21) proximal zum Vorsprung (16) angeordnet wird, die wenigstens einen Teilbereich des zweiten Abschnitts (B2) und im Wesentlichen den gesamten Bereich des vierten Abschnitts (B4) umgreift und/oder dass durch die erste Crimphülse (20), die zweite Crimphülse (21), das Eingriffselement (12) sowie das Kopplungsstück (14) im zusammengebauten Zustand eine Reihenanordnung (R) gebildet wird, die mit einem 0,017"-Kather mit einem Innenlumen von 0,420 mm passgenau kompatibel ist.

## Claims

1. A medical device, in particular a thrombectomy device for intravascular treatment, with a lattice structure (10) which is tubular at least in sections and formed from webs (28) which are connected together into one piece, wherein the lattice structure (10) has a web extension (11) at a proximal end section (10b) thereto, which comprises an engaging element (12), wherein the engaging element (12) is connected to a distal coupling piece (14) of a transport wire (15) by an interlocking connection (13) and the coupling piece (14) has at least one projection (16) and the engaging element (12) has at least one recess (17), wherein the projection (16) engages in the recess (17) of the engaging element (12),
**characterized in that**
the recess (17) is formed in a closed manner, wherein the recess extends substantially in the axial direction of the engaging element (12).

2. The medical device as claimed in claim 1,
**characterized in that**
the recess (17) substantially corresponds to the contour of the engaging element (12).

3. The medical device as claimed in claim 1 or claim 2,
**characterized in that**
the coupling piece (14) has at least two projections (16) which are configured as a transverse anchor (18).

4. The medical device as claimed in one of the preceding claims,
**characterized in that**
the transverse anchor (18) is configured as a T-anchor.

5. The medical device as claimed in one of the preceding claims,
**characterized in that**
the shape of the projection (16) corresponds to that of the recess (17), in particular wherein the projection (16) extends substantially in the axial direction of the coupling piece (14).

6. The medical device as claimed in one of the preceding claims,
**characterized in that**
the coupling piece (14) comprises a plurality of axially adjacent sections (B1-B5) with respectively different outer diameters, wherein the projection (16) has the maximum outer diameter, in particular wherein a first section (B1) is disposed immediately distally to the projection (16) and a second section (B2) is disposed immediately proximally to the projection (16).

7. The medical device as claimed in claim 6,
**characterized in that**
a third section (B3) is disposed immediately distally to the first section (B1) and/or **in that** a fourth section (B4) is disposed immediately proximally to the second section (B2), in particular wherein a fifth section (B5) is disposed immediately proximally to the fourth section (B4), wherein the fifth section (B5) has a larger outer diameter than the fourth section (B4), in particular wherein the fifth section (B5) has a taper (19) which extends in the proximal direction.

8. The medical device as claimed in claim 6 or claim 7,
**characterized in that**
at least one first crimp sleeve (20) is provided which is disposed distally to the projection (16) and encompasses at least a subregion of the first section (B1) and substantially the entire region of the third section (B3).

9. The medical device as claimed in one of claims 6 to 8,
**characterized in that**
at least one second crimp sleeve (21) is provided which is disposed proximally to the projection (16) and which encompasses at least one subregion of the second section (B2) and substantially the entire region of the fourth section (B4).

10. The medical device as claimed in one of the preceding claims,
**characterized in that**
in the assembled state, the first crimp sleeve (20), the second crimp sleeve (21), the engaging element (12) as well as the coupling piece (14) form a sequential arrangement (R), in particular wherein the sequential arrangement (R) is exactly compatible with a 0.017" catheter with an inner lumen of 0.420 mm.

11. The medical device as claimed in one of the preceding claims,
**characterized in that**
the engaging element (12) has a greater width than the web extension (11), in particular wherein the cross section of the engaging element (12) is formed trapezoidal, wherein the wider side (22) of the trapezoid (T) is disposed on the inside of the engaging element (12), in particular wherein, in the region of the recess (17), the engaging element (12) has two parallel sides (S1, S2) which extend in the axial direction of the engaging element (12).

12. The medical device as claimed in one of claims 8 to 11,
**characterized in that**
at least one radiopaque wire element (24) is provided which is guided along at least one web, wherein the wire element (24) has a wire end (23) which is guided through the first crimp sleeve (20), in particular wherein the wire end (23), the first crimp sleeve (21) and the second crimp sleeve (22) are connected to the coupling piece (14) by adhesive.

13. A method for the manufacture of a medical device as claimed in one of the preceding claims, wherein
- a projection (16), in particular a transverse anchor (18), is manufactured by laser material processing, wire erosion or grinding at a distal coupling piece (14) of a transport wire (15);
- a recess (17) is manufactured in an engaging element (12) of a web extension (11) by a laser cutting process, wherein the recess (17) extends substantially in the axial direction of the engaging element (12);
- the engaging element (12) is interlockingly connected to the coupling piece (14), wherein the projection (16) engages in at least one recess (17) of the engaging element (12);
- at least one first crimp sleeve (20) is disposed distally to the projection (16) and encompasses at least one subregion of the first section (B1) and substantially the entire region of the third section (B3).

14. The method as claimed in claim 13,
**characterized in that**
at least one second crimp sleeve (21) is disposed proximally to the projection (16) and encompasses at least one subregion of the second section (B2) and substantially the entire region of the fourth section (B4) and/or **in that** in the assembled state, a sequential arrangement (R) is formed by the first crimp sleeve (20), the second crimp sleeve (21), the engaging element (12) as well as the coupling piece (14) which is exactly compatible with a 0.017" catheter with an inner lumen of 0.420 mm.

## Revendications

1. Dispositif médical, en particulier dispositif de thrombectomie, destiné au traitement intravasculaire, comportant une structure grillagée (10) de forme tubulaire au moins par sections et composée de barrettes (28) connectées en une pièce les unes aux autres, la structure grillagée (10) présentant, sur une section terminale proximale (10b), un prolongement de barrette (11) qui comprend un élément d'engrènement (12), l'élément d'engrènement (12) étant connecté par une connexion en correspondance géométrique (13) à une pièce de couplage distale (14) d'un câble de transport (15) et la pièce de couplage (14) présentant au moins une saillie (16) et l'élément d'engrènement (12) au moins un évidement (17), la saillie (16) s'engrenant dans l'évidement (17) de l'élément d'engrènement (12),
**caractérisé en ce que**
l'évidement (17) a une conformation fermée, l'évidement s'étendant sensiblement dans le sens axial de l'élément d'engrènement (12).

2. Dispositif médical selon la revendication 1,
**caractérisé en ce que**
l'évidement (17) correspond sensiblement au contour de l'élément d'engrènement (12).

3. Dispositif médical selon la revendication 1 ou 2,
**caractérisé en ce que**
la pièce de couplage (14) présente au moins deux saillies (16) qui sont réalisées sous forme d'ancrages transversaux (18).

4. Dispositif médical selon une des revendications précédentes,
**caractérisé en ce que**
l'ancrage transversal (18) est réalisé sous forme d'un ancrage en T.

5. Dispositif médical selon une des revendications précédentes,
**caractérisé en ce que**
la saillie (16) correspond à la forme de l'évidement (17), en particulier la saillie (16) s'étendant sensiblement dans le sens axial de la pièce de couplage (14) .

6. Dispositif médical selon une des revendications précédentes,
**caractérisé en ce que**
la pièce de couplage (14) présente plusieurs sections axialement voisines (B1-B5) qui présentent respectivement différents diamètres extérieurs, la saillie (16) présentant le diamètre extérieur maximal, une première section (B1) étant en particulier disposée directement distalement et une deuxième section (B2) directement proximalement par rapport à la saillie (16) .

7. Dispositif médical selon la revendication 6,
**caractérisé en ce**
**qu'**une troisième section (B3) est disposée directement distalement par rapport à la première section (B1) et/ou qu'une quatrième section (B4) est disposée directement proximalement par rapport à la deuxième section (B2), en particulier une cinquième section (B5) étant disposée directement proximalement par rapport à la quatrième section (B4), la cinquième section (B5) présentant un diamètre extérieur supérieur à celui de la quatrième section (B4), en particulier la cinquième section (B5) présentant un rétrécissement (19) qui s'étend dans le sens proximal.

8. Dispositif médical selon la revendication 6 ou 7,
**caractérisé en ce**
**qu'**il est prévu au moins un premier manchon de sertissage (20) qui est disposé distalement par rapport à la saillie (16) et entoure au moins une zone partielle de la première section (B1) et sensiblement l'ensemble de la zone de la troisième section (B3).

9. Dispositif médical selon une des revendications 6 à 8,
**caractérisé en ce**
**qu'**il est prévu au moins un second manchon de sertissage (21) qui est disposé proximalement par rapport à la saillie (16) et entoure au moins une zone partielle de la deuxième section (B2) et sensiblement l'ensemble de la zone de la quatrième section (B4).

10. Dispositif médical selon une des revendications précédentes,
**caractérisé en ce que**
le premier manchon de sertissage (20), le second manchon de sertissage (21), l'élément d'engrènement (12) et la pièce de couplage (14), en état assemblé, forment un agencement en rangée (R), en particulier l'agencement en rangée (R) étant parfaitement compatible avec un cathéter de 0,017" ayant un lumen intérieur de 0,420 mm.

11. Dispositif médical selon une des revendications précédentes,
**caractérisé en ce que**
l'élément d'engrènement (12) présente une largeur supérieure à celle du prolongement de barrette (11), la section transversale de l'élément d'engrènement (12) étant en particulier réalisée en forme trapézoïdale, le côté large (22) du trapèze (T) étant disposé sur la face intérieure de l'élément d'engrènement (12), l'élément d'engrènement (12) présentant en particulier au niveau de l'évidement (17) deux faces parallèles (S1, S2) qui s'étendent dans le sens axial de l'élément d'engrènement (12).

12. Dispositif médical selon une des revendications 8 à 11,
**caractérisé en ce**
**qu'**il est prévu au moins un élément filaire (24) visible par radiographie qui est guidé le long d'au moins une barrette, l'élément filaire (24) présentant une extrémité de fil (23) qui passe à travers le premier manchon de sertissage (20), en particulier l'extrémité de fil (23), le premier manchon de sertissage (21) et le second manchon de sertissage (22) étant connectés à la pièce de couplage (14) par collage.

13. Procédé de fabrication d'un dispositif médical selon une des revendications précédentes, dans lequel
- une saillie (16), en particulier un ancrage transversal (18), est fabriquée par un usinage de matériau au laser, une érosion par fil ou un meulage au niveau d'une pièce de couplage distale (14) d'un câble de transport (15) ;
- un évidement (17) est fabriqué dans un élément d'engrènement (12) d'un prolongement de barrette (11) par un procédé de coupe au laser, l'évidement (17) s'étendant sensiblement dans le sens axial de l'élément d'engrènement (12) ;
- l'élément d'engrènement (12) est connecté en correspondance géométrique avec la pièce de couplage (14), la saillie (16) s'engrenant dans au moins un évidement (17) de l'élément d'engrènement (12) ;
- au moins un premier manchon de sertissage (20) est disposé distalement par rapport à la saillie (16), lequel manchon de sertissage entoure au moins une zone partielle de la première section (B1) et sensiblement l'ensemble de la zone de la troisième section (B3).

14. Procédé selon la revendication 13,
**caractérisé en ce**
**qu'**au moins un second manchon de sertissage (21) est disposé proximalement par rapport à la saillie (16), lequel manchon de sertissage entoure au moins une zone partielle de la deuxième section (B2) et sensiblement l'ensemble de la zone de la quatrième section (B4) et/ou qu'est constitué par le premier manchon de sertissage (20), le second manchon de sertissage (21), l'élément d'engrènement (12) et la pièce de couplage (14), en état assemblé, un agencement en rangée (R) qui est parfaitement compatible avec un cathéter de 0,017"' ayant un lumen intérieur de 0,420 mm.
